# EUROPEAN PATENT SPECIFICATION

(11) **EP 4 042 165 B1**
(45) Date of publication and mention of the grant of the patent: **15.11.2023**
(21) Application number: 20789936.0
(22) Date of filing: 09.10.2020
(51) Int. Cl.: G01N 33/68

(54) **2-METHYLBUTYRATE, A BIOMARKER FOR IMPROVEMENTS OF MOOD DISORDERS**
2-METHYLBUTYRAT, EIN BIOMARKER ZUR VERBESSERUNG VON GEMÜTSKRANKHEITEN
2-MÉTHYLBUTYRATE, UN BIOMARQUEUR POUR L'AMÉLIORATION DES TROUBLES DE L'HUMEUR

(30) Priority: 11.10.2019 EP 19202842
(43) Date of publication of application: 17.08.2022
(73) Proprietor: Société des Produits Nestlé S.A., 1800 Vevey (CH)
(72) Inventor: MARTIN, François-Pierre, 1687 Vuisternens-devant-Romont (CH); COMINETTI ALLENDE, Ornella, 1026 Denges (CH); BERGONZELLI DEGONDA, Gabriela, 1030 Bussigny (CH)
(74) Representative: Chautard, Cécile
(86) International application number: PCT/EP2020/078389
(87) International publication number: WO 2021/069650

(56) References cited:
- EP-A1- 2 466 312
- EP-A1- 3 351 934
- ASMA KAZEMI ET AL: "Effect of probiotic and prebiotic vs placebo on psychological outcomes in patients with major depressive disorder: A randomized clinical trial", CLINICAL NUTRITION., vol. 38, no. 2, 1 April 2019 (2019-04-01), pages 522-528, XP055647617, GB ISSN: 0261-5614, DOI: 10.1016/j.clnu.2018.04.010

## Description

The present invention relates generally to the field of biomarkers. The biomarker identified in the present invention is 2-methylbutyrate. For example, the present invention relates to the use of 2-methylbutyrate as a biomarker for detecting and/or quantifying improvements of the mood disorder status and/or the resulting emotional reaction of a subject. Embodiments of the resent invention relate to a method of detecting and/or quantifying improvements of mood disorder status and/or the resulting emotional reaction of a subject, comprising determining the level of 2-methylbutyrate in a body sample obtained from a subject to be tested, and comparing the subject's 2-methylbutyrate level to a predetermined reference value, wherein an increased 2-methylbutyrate level in the sample compared to the predetermined reference value indicates an improvement of the mood disorder status and/or the resulting emotional reaction of the subject.

According to a fact sheet published by the World Health Organization (WHO) in 2018, more than 300 million people worldwide suffer from depression. Among them, depression is a common illness that is different from normal changes in mood and short-lived emotional responses to challenges in everyday life. However, subclinical depression, a milder mood disorder may also affect quality of life.

Mood disorders can have severe effects for the concerned individual and for the persons the affected individual is interacting with on a regular basis. Typical consequences are poor performance at work or in school, decreased social interaction, personal suffering and a negative influence on relationships with friends or family.

In the worst case, mood disorders can lead to suicide. Close to 800 000 people die due to suicide every year with suicide being the second leading cause of death in 15-29-year-olds.

Mood disorders appear to be more prevalent in women than in men (Journal of the American Medical Association, 2003; Jun 18; 289(23): 3095-105); perinatal period, and post-menopause being particular susceptible moments. Also, 1.9 million children are diagnosed with depression. Notably, mood disorders may also lead to other diseases later on. It is known, for example, that mood disorders result in a greater risk to develop coronary artery disease.

Mood disorders can usually be treated successfully today. For example, mood disorders can be treated by exercise or talking therapy, ideally guided by a psychologist. Psychotherapy, for example a cognitive behavioral therapy, is an option. As medicaments antidepressants are used successfully today. Often combinations of the above referenced approaches are used in the framework of a combination therapy. Recent scientific work has revealed that the probiotic *Bifidobacterium longum* NCC3001 reduces depression scores (Gastroenterology 2017;153:448-459) in patients with irritable bowel syndrome.

Doctors diagnose mood disorders today by talking with the patient and by screening for typical symptoms. Treatment responses should be measured today by systematically monitoring patients' responses using validated self-rated scales (J Clin Psychiatry. 2013 Jul;74(7)).

It would be desirable to have available a biochemical tool that allows it to detect mood disorders and/or to assess the success of a therapy to treat or ameliorate mood disorders.

The present inventors have addressed this need.

Any reference to prior art documents in this specification is not to be considered an admission that such prior art is widely known or forms part of the common general knowledge in the field.

The objective of the present invention was it, hence, to improve the state of the art and in particular to provide a biochemical tool that allows it to diagnose mood disorders or improvements of the mood disorder status and/or the resulting emotional reaction of a subject, or to at least provide a useful alternative.

The inventors were surprised to see that the objective of the present invention could be achieved by the subject matter of the independent claims. The dependent claims further develop the idea of the present invention.

Accordingly, the present invention provides a use of 2-methylbutyrate as a biomarker for detecting and/or quantifying improvements of the mood disorder status and/or the resulting emotional reaction of a subject.

Finally, the present invention provides a method for detecting and/or quantifying mood disorders, improvements of the mood disorder status and/or the resulting emotional reaction of a subject, comprising determining the level of 2-methylbutyrate in a body sample obtained from a subject to be tested, and comparing the subject's 2-methylbutyrate level to a predetermined reference value, wherein an increased 2-methylbutyrate level in the sample compared to the predetermined reference value indicates an improvement of the mood disorder status and/or the resulting emotional reaction of the subject.

As used in this specification, the words "comprises", "comprising", and similar words, are not to be interpreted in an exclusive or exhaustive sense. In other words, they are intended to mean "including, but not limited to".

The present inventors have shown that 2-methylbutyrate can be used as a biomarker for detecting and/or quantifying improvements of the mood disorder status and/or the resulting emotional reaction of a subject.

For the purpose of the present invention the term "mood disorder" shall be understood to include depression, such as severe depression like major depression disorders and subclinical depression which is a mild to moderate mood disorder.

In a prospective, randomized placebo-controlled, double blind, multicenter study subjects, men and women aged 21-65, with symptoms of anxiety and depression, were treated with *Bifidobacterium longum* to treat or ameliorate depression. Biospecimens of human urine, blood, and feces collected during the clinical trial were subjected to metabonomics analysis. Metabolite data were analysed using multivariate data modelling approaches to identify signatures associated with treatment and improvement of clinical parameters. An analysis of the biospecimens revealed that probiotic supplementation resulted in increase in the circulation of the metabolite 2-methylbutyrate correlating with an improvement in depression score.

Without wishing to be bound by theory, the inventors presently believe that circulating 2-methylbutyrate might be a readout indicative of a shift in protein and aromatic amino acid metabolism by the gut microbiota, and therefore might directly or indirectly describe probiotic-induced gut-brain metabolic interactions associated with the improvement of the mood disorder status.
Figure 1 shows metabolite concentrations in ng per mL in blood. Data are reported as mean and standard error.
Figure 2: Overview of spearman correlation coefficient plots between plasma metabolites and depression score variations.

Table 1 shows a list of metabolites detected and quantified in the human samples using MS-based metabonomics (Hawaii Cancer Research Center)

Biomarkers are well known to people skilled in the art. They are usually understood as a characteristic that is objectively measured and evaluated as an indicator of normal biological processes, pathogenic processes, or responses to an intervention. Further guidance can be obtained from Curr Opin HIV AIDS. 2010 Nov; 5(6): 463-466.

The present invention relates to the use of 2-methylbutyrate (sometimes referred to as ethylmethylacetate) as a biomarker for detecting and/or quantifying improvements of the mood disorder status and/or the resulting emotional reaction of a subject. Accordingly, 2-methylbutyrate may be used as a biomarker for detecting mood disorders. 2-Methylbutyrate may further be used for detecting and/or quantifying improvements of the mood disorder status. 2-Methylbutyrate may further be used for detecting and/or quantifying improvements of the emotional reaction of a subject resulting from its mood disorder status. For example, the authors of Gastroenterology 2017;153:448-459 describe that a change in engagement of the amygdala correlated with a change in mood disorder scores. The amygdala plays a primary role in emotional responses, so that it can be concluded that an improvement of the mood disorder status will correspond to an improvement of the emotional reaction of a subject resulting from its mood disorder status.

The subject matter of the present invention further relates to a method for detecting and/or quantifying improvements of the mood disorder status and/or the resulting emotional reaction of a subject, comprising
- determining the level of 2-methylbutyrate in a body sample obtained from a subject to be tested, and
- comparing the subject's 2-methylbutyrate level to a predetermined reference value,
wherein an increased 2-methylbutyrate level in the sample compared to the predetermined reference value indicates an improvement of the mood disorder status and/or the resulting emotional reaction of the subject.

The subject matter of the present invention further relates to a method for detecting mood disorders in a subject, comprising
- determining the level of 2-methylbutyrate in a body sample obtained from a subject to be tested, and
- comparing the subject's 2-methylbutyrate level to a predetermined reference value,
wherein a decreased 2-methylbutyrate level in the sample compared to the predetermined reference value indicates the mood disorder in the subject.

The method of the present invention has the advantage that it allows to diagnose mood disorders based on the concentration of a biomarker or the change of the concentration of a biomarker in a body sample. It also allows to control the success of a treatment of mood disorders in a subject. Such a biochemical method can, hence, be a valuable tool to assist doctors in diagnosing mood disorders and/or to follow the success of the treatment they prescribe, while they would otherwise largely have to rely on questionnaires and the patient's description of their symptoms, only. Also, the method of the present invention will be very valuable to help subjects that are unable to communicate clearly and suffer from mood disorders, for example companion animals.

The method of the present invention compares a level of 2-methylbutyrate in a body sample obtained from a subject to be tested with a reference value.

For example, when aiming to detecting and/or quantifying improvements of the mood disorder status and/or the resulting emotional reaction of a subject, it may be preferred if the reference value was also obtained from the subject to be treated.

Hence, for the method of the present invention, the predetermined reference value may have been obtained previously from the same subject. This has the advantage that an increase of the 2-methylbutyrate level can be reliably measured for an individual by comparing the current 2-methylbutyrate level to a previous 2-methylbutyrate level.

Alternatively, the predetermined reference value may be based on an average 2-methylbutyrate level in the same body sample in a control population. This has the advantage that the measured 2-methylbutyrate level of an individual can be compared to a standard that is generally applicable, so that the 2-methylbutyrate level of an individual can be compared to a general average. This allows for an easy comparison of many measurements in many individual patients. It also allows for a quick assessment by making one test only, as there is no need for a previous test to obtain an individual reference value.

The analysis of the 2-methylbutyrate level in the body sample can be carried out by any suitable method known to the person skilled in the art. The present inventors have used mass spectrometry. Hence, in one embodiment of the present invention, the level of the biomarker in the sample and in the reference may be determined by mass spectrometry. For an increased speed, accuracy and reduction of noise, mass spectrometry may be coupled with a chromatographic step preceding the mass spectrometry. For example, the level of the biomarker in the sample and in the reference may be determined by ultra-performance liquid chromatography coupled to tandem mass spectrometry. Further, for example the level of the biomarker in the sample and in the reference may be determined by gas chromatography coupled to tandem mass spectrometry. For example, the quantitative measurement of the 2-methylbutyrate level in samples may be carried out using both ultra-performance liquid chromatography coupled to tandem mass spectrometry (UPLC-MS/MS) and/or gas chromatography time-of-flight mass spectrometry (GC-TOFMS).

Advantageously, in order to ensure optimal comparability of reference value and the 2-methylbutyrate level obtained from the body sample, both, the reference value and the present 2-methylbutyrate level may be obtained from the same body sample. Hence, the predetermined reference value may be based on a 2-methylbutyrate level obtained from the same body sample as the level of 2-methylbutyrate in a body sample obtained from a subject to be tested.

The method of the present invention may be used to monitor the success of a mood disorder treatment. In order to do this, it may be preferred to be able to compare current 2-methylbutyrate levels to a 2-methylbutyrate level obtained from the subject who is being treated before the treatment was started. Hence, for example, the subject's predetermined reference value may be obtained from a body sample that was collected from the subject before an intervention to treat or ameliorate a mood disorder status and/or the resulting emotional reaction started.

In order to be able to assess further improvements in mood disorder status after an invention has already started, it may still be preferred to have available a reference obtained from the subject that is being treated. Hence, for example, the subject's predetermined reference value may be obtained from a body sample that was collected from the subject during an intervention to treat or ameliorate a mood disorder status and/or the resulting emotional reaction, but at least one week, for example at least two weeks, at least four weeks, or at least six weeks, before the body sample is obtained from the subject. This has the advantage that a continuous progress in the treatment of the mood disorder can continuously be monitored.

Generally, any increase in the detected 2-methylbutyrate level indicates an improvement of the mood disorder status and/or the resulting emotional reaction of the subject. However, one advantage of the biomarker of the present invention is that the differences in biomarker concentration in the body sample that can be measured in a successful treatment are rather pronounced. Hence, for example, in the method of the present invention an increase in the 2-methylbutyrate level in the sample compared to the predetermined reference value of at least 10%, at least 20%, at least 30%, at least 40%, or at least 50% , or at least 60% indicates an improvement of the mood disorder status and/or the resulting emotional reaction of the subject.

The present inventors have found that typical body samples that may be used for the purpose of the present invention may be selected from the group consisting of feces, urine, blood, blood serum, and blood plasma.

Advantageously, both reference and the current 2-methylbutyrate level are both obtained from the same body sample, for example, both reference and the current 2-methylbutyrate level are both obtained from feces, both reference and the current 2-methylbutyrate level are both obtained from urine, both reference and the current 2-methylbutyrate level are both obtained from blood, both reference and the current 2-methylbutyrate level are both obtained from blood serum, or both reference and the current 2-methylbutyrate level are both obtained from blood plasma.

For example, from urine, blood, blood serum, or blood plasma about 5 -10 ml may be collected. A large enough sample size avoids that artifacts are generated. From these samples, about 20 -100 µl may be used for further analysis. From feces about 5 - 10 g may be collected. 2-10 mg of the stool sample may then be used for further analysis.

Blood, blood serum and/or blood plasma have the advantage that the signal to noise ratio for the biomarker to be tested is particularly high. Urine or feces have the advantage that the body fluid sample can be obtained non-invasively. Irrespective of the chosen body sample, the method of the present invention has the advantage that obtaining such body fluids from a subject is a well-established procedure. The actual diagnosis method is then carried out in a body sample outside the body.

The method of the present invention is suitable to monitor the progress of any treatment of mood disorders. For example, the mood disorder treatment may be selected from the group consisting of exercise, talking therapy, psychotherapy, cognitive behavioral therapy, antidepressant administration, nutritional intervention for example with probiotics, and combinations thereof.

Probiotics have been found to have an effect on the symptoms of depression (Neuropsychobiology. 2019 Feb 13:1-9. doi: 10.1159/000496406) (Gastroenterology 2017;153:448-459). These probiotics can help to treat a range of mental health conditions, including mood disorders. Without wishing to be bound by theory, the present inventors currently believe that this effect is seen, because of the gut-brain axis, a strong connection between gastrointestinal tract and brain. Hence, in one embodiment of the present invention, the method is for monitoring the progress of an intervention to treat or ameliorate a mood disorder status and/or the resulting emotional reaction in a subject, wherein the intervention comprises the administration of a probiotic.

The present inventors have carried out the studies presented herein using an intervention with the probiotic *Bifidobacterium longum* NCC3001 as an example. Consequently, for the purpose of the present invention the probiotic may be *Bifidobacterium longum,* for example *Bifidobacterium longum* NCC3001.

Also companion animals can suffer from mood disorders. For the purpose of the present invention, a companion animal is an animal kept primarily for a person's company, entertainment or as an act of compassion. Typical examples for companion animals are cats or dogs; but also rabbits; ferrets; pigs; rodents, such as gerbils, hamsters, chinchillas, rats, mouse and guinea pigs; or birds. When, for example, dogs are depressed, they often appear withdrawn, lose interest to play, and/or appear lethargic or sad. Sometimes, they will eat and/or drink less than usual which might result in a variety of physical illnesses. As a result, today also companion animals are treated for mood disorders. Hence, in one embodiment of the present invention, the subject may be a human or a companion animal such as a cat or a dog.

### Examples:

### Example 1

### Methods used during the study

### Experimental design

This is a prospective, randomized placebo-controlled, double blind, multicenter study with 2 groups in parallel carried out at McMaster University Medical Center, Hamilton, Canada.

In each study group, the number of subjects to complete the study protocol was 20, and subjects were randomly assigned to one of the treatment groups, namely product containing B. longum or placebo, without B. longum.

The baseline period lasted 4 weeks. Patients then received the treatment for 6 weeks and were followed-up for another 4 weeks.

Men and women aged 21-65 with mixed or diarrhoea-predominant IBS based on Rome III criteria and with symptoms of anxiety and depression were enrolled into the study according to defined inclusion and exclusion criteria (study protocol 09.25.NRC).

### Sample collection

Blood plasma, stool and urine samples were collected for metabolomics at end of the baseline period and at the end of the treatment period. Blood samples were obtained in EDTA-anticoagulated tubes aliquots of 500 uL were transferred into cryotubes and stored at -80°C prior to analysis. Stool samples were collected at home by the patients and stored at -80°C prior to analysis. Samples were freeze-dried and weighted. Aliquots of 5/6 mg were then transfered into cryotubes and stored at -80°C. Morning urine samples (5-10 mL) were collected by means of a sterile labelled plastic tubes. Antibacterial agent (sodium azide 1mM) was added to samples prior to transfer into 1mL cryotubes and storage at -40°C.

### Metabolomic analysis

### Chemicals

All standards were obtained from Sigma-Aldrich and Nu-Chek Prep (Elysian, MN, USA). The stock solutions of all reference standards were prepared in methanol of HPLC grade or ultrapure water with the concentration of 5 mg mL-1 or 1 mg mL-1 and stored at -80 _{°}C until use. The mixed standard solutions containing methanol-soluble or watersoluble standards were prepared by combining the standards with the same chemical class. Totally, ^{~}150 representative compounds from different chemical classes (amino acids, fatty acids, carboxylic acids, hydroxyl acids, phenolic acids, indoles, etc.) were chosen, and a series standard solutions were made by diluting the mixed standard solutions to build the calibration curves. A mixture of internal RI markers was prepared using thirteen normal alkanes of C8, C9, C10, C12, C14, C16, C18, C20, C22, C24, C26, C28, and C30 linear chain length, dissolved in chloroform respectively at a concentration of 5 mg mL-1.

### Preparation of biological samples

Following protein precipitation, dried samples were sealed and stored at -80 °C for subsequently automated alkylchloroformate derivatization before gas chromatography (GC) mass spectrometry analysis (MS), using a GC (Agilent 6890N), coupled with a time-of-flight MS (LECO Pegasus HT). GC-MS data were exported in NetCDF format to the software ChromaTOF (v4.50, Leco Co., CA, USA) for processing and metabolite identification. Compound identification was performed by comparing both MS and Kovats-RI with reference standards in author-constructed two alkyl chloroformates derivatives databases, with a similarity of more than 70%. The data sets were exported in CSV file that included sample name, compound name, Kovats-RI, quantification mass, peak area and concentration.

### Statistical analysis

Chemometric analysis was performed using the software package SIMCA-P+ (version 14.0, Umetrics AB, Umeå, Sweden) and in-house developed MATLAB routines in order to detect the presence of inherent similarities between metabolic profiles. In order to detect the presence of similarities between metabolic profiles, Principal Component Analysis (PCA), Projection to Latent Structure (PLS) and the Orthogonal Projection to Latent Structures (O-PLS) were used. Seven-fold cross validation was used to assess the validity of the model. The classification accuracy of the O-PLS-DA model was established from the predicted samples in the 7-fold cross-validation cycle. Influential MS based metabolite concentrations were analyzed using Wilcoxon-Mann-Whitney tests.

### Example 2

### Measurement of metabolite concentrations

The targeted and quantiative GC MS analysis provides quantitative measure for about 100 metabolites in the three matrices. A total of 56 metabolites was quantified in human blood samples, 74 in human urine samples, and 85 in human fecal samples (Table 1). The method has yielded good quality control results to ensure the quality of the data generated with regard to metabolites. Using the methods developed for this project, a majority of previously reported gut microbiome metabolites have been covered in the measurement. Such a dataset rich in microbiome products allowed us to comprehensively examine human pathophysiology through the metabolic variations of the gut microbiota and host metabolites.

### Example 3

### Metabolites associated with probiotic induced improvement of mood disorders

For MS metabonomics data analysis, the urine, stool and plasma samples from 19 patients being assigned to the placebo group, and 18 patients to the probiotic treatment group were subjected to multivariate data analysis. OPLS discriminant analysis (OPLS-DA) was applied using one predictive and two orthogonal components at baseline and post-intervention to model metabolic differences between the placebo and the probiotics groups.

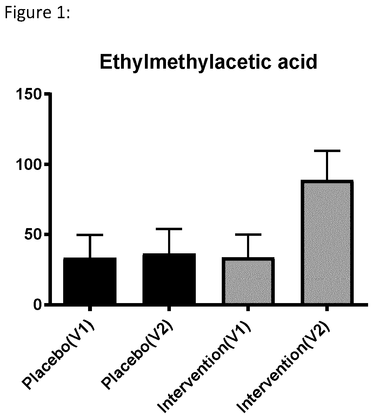

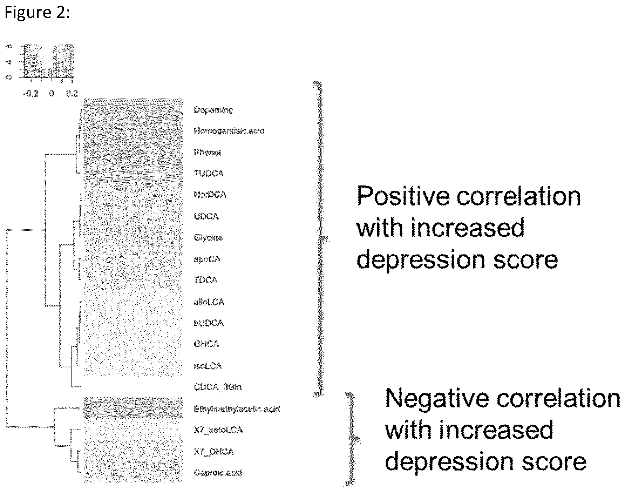

A statistically robust multivariate model could be obtained based on the plasma metabolic profiles post-intervention, discriminating control subjects from the patients supplemented with the probiotics (model parameters: (R2X 0.35, R2Y 0.86, Q2Y 0.11). Metabolites contributing to the group separation were identified through analysis of the OPLS-DA coefficients plots. Statistical significant differences were tested using Wilcoxon-Mann-Whitney tests as a way to indicate the most significant metabolite pattern from a univariate data analysis stand-point. The supplementation of Bifidobacterium longum NCC3001 could be particularly ascribed to a shift in the concentration of ethylmethylacetate (or 2methylbutyrate) and phenol (Figures 1 and, 2).

Additional PLS regression analyses were applied to explore relationships between urine, blood plasma and stool metabolite composition and clinical endpoints related to the improvement in mood disorders, as measured through improvement in depression. Benefiting from targeted and quantitative MS metabolite concentrations, multivariate models explored the relationships between fold of changes in metabolites and variations in depression scores overtime. Based on the most influential metabolites identified in the multivariate models, spearman correlations were further tested (Figure 4).

Amongst the most significant association, improvement in depression was associated with increased plasma ethylmethylacetate (2-methylbutyrate), and decreased plasma phenol, which were previously shown to be influenced by the probiotic supplementation. In addition, Improvement of depression was associated to decreased dopamine, homogentisic acid, and glycine, three key metabolites involved in neurotransmitter metabolism. In urine, improvement in depression was associated with increased urinary level in gamma aminobutyratic acid (GABA), tryptophan, N-acetyltryptophan, which are also key metabolites involved in neurotransmitter metabolism.

The probiotic supplementation induced an increased circulation of 2-methylbutyrate, but a decrease in phenol, which is an effect more likely mediated through a modulation of the gut microbial functional ecology. 2-methylbutyrate (increased with probiotics) appeared to correlate positively with depression improvement and phenol (decreased with probiotics) to correlate negatively with depression improvement. Both metabolites are readouts indicative of a shift in protein and aromatic amino acid metabolism by the gut microbiota, and therefore may directly or indirectly describe probiotic-induced gut-brain metabolic interactions associated with the improvement of depression status.

Phenol has been studied and showed similar properties to inhibit dopamine beta-hydroxylase (Kim, S.C.; Klinman, J.P. Mechanism of inhibition of dopamine beta-monooxygenase by quinol and phenol derivatives, as determined by solvent and substrate deuterium isotope effects. Biochemistry 1991, 30, 8138-8144). This metabolite may be involve in the mechanism of action of probiotic-related improvement in depression metabolism. The phenol pattern is similar in blood, urine and stool.

**Table 1:**

| No. | **HMDB ID (when available)** | Analytes | **FECES/5^{~}6** mg | Plasma/100 **µl** | **URINE/100 µl** |
|---|---|---|---|---|---|
| 1 | HMDB00237 | Propionic acid | X | | |
| 2 | HMDB01873 | Isobutyric acid | X | X | X |
| 3 | HMDB00039 | Butyric acid | X | X | X |
| 4 | HMDB00718 | Isovaleric acid | X | X | X |
| 5 | HMDB02176 | Ethylmethylacetic acid | X | X | X |
| 6 | HMDB00892 | Valeric acid | X | X | X |
| 7 | HMDB02329 | Oxalic acid | X | | X |
| 8 | HMDB31580 | 2-Methylpentanoic acid | | X | |
| 9 | HMDB00754 | 3-Hydroxyisovaleric acid | | | X |
| 10 | HMDB33774 | 3-Methylpentanoic acid | X | | |
| 11 | HMDB00535 | Caproic acid | | X | X |
| 12 | HMDB00691 | Malonic acid | X | | |
| 13 | HMDB00243 | Pyruvic acid | X | | X |
| 14 | HMDB00115 | Glycolic acid | X | | |
| 15 | HMDB00666 | Heptanoic acid | X | X | |
| 16 | HMDB00134 | Fumaric acid | X | | |
| 17 | HMDB00254 | Succinic acid | X | X | X |
| 18 | HMDB00190 | L-Lactic acid | X | X | X |
| 19 | HMDB00729 | Alpha-Hydroxyisobutyric acid | | | X |
| 20 | HMDB01844 | Methylsuccinic acid | X | | X |
| 21 | HMDB00073 | Dopamine | X | X | X |
| 22 | HMDB00068 | Epinephrine | X | X | X |
| 23 | HMDB02092 | Itaconic acid | X | | X |
| 24 | HMDB00634 | Citraconic acid | X | | X |
| 25 | HMDB00008 | 2-Hydroxybutyric acid | X | X | X |
| 26 | HMDB00426 | Citramalic acid | X | | X |
| 27 | HMDB00482 | Octanoic acid | X | X | X |
| 28 | HMDB00123 | Glycine | X | X | X |
| 29 | HMDB00161 | L-Alanine | X | X | X |
| 30 | HMDB00661 | Glutaric acid | X | | |
| 31 | HMDB00357 | 3-Hydroxybutyric acid | X | X | X |
| 32 | HMDB01488 | Nicotinic acid | X | | |
| 33 | HMDB00228 | Phenol | X | X | X |
| 34 | HMDB00209 | Phenylacetic acid | X | | X |
| 35 | HMDB00452 | L-Alpha-aminobutyric acid | X | X | X |
| 36 | HMDB00056 | Beta-Alanine | X | | |
| 37 | HMDB00847 | Nonanoic acid | X | X | X |
| 38 | HMDB00448 | Adipic acid | X | | X |
| 39 | HMDB03911 | 3-Aminoisobutanoic acid | | | X |
| 40 | HMDB02048 | m-Cresol | X | | X |
| 41 | HMDB01858 | p-Cresol | X | | X |
| 42 | HMDB00883 | L-Valine | X | X | X |
| 43 | HMDB00208 | Oxoglutaric acid | X | X | X |
| 44 | HMDB00764 | Hydrocinnamic acid | X | | |
| 45 | HMDB00112 | Gamma-Aminobutyric acid | X | | |
| 46 | HMDB00857 | Pimelic acid | | | X |
| 47 | HMDB00687 | L-Leucine | X | X | X |
| 48 | HMDB00172 | L-Isoleucine | X | X | X |
| 49 | HMDB00744 | Malic acid | X | X | X |
| 50 | HMDB00267 | Pyroglutamic acid | X | X | X |
| 51 | HMDB00162 | L-Proline | X | X | X |
| 52 | HMDB00168 | L-Asparagine | | X | X |
| 53 | HMDB00072 | cis-Aconitic acid | X | X | X |
| 54 | HMDB00707 | 4-Hydroxyphenylpyruvic acid | X | | |
| 55 | HMDB00893 | Suberic acid | X | X | X |
| 56 | HMDB00139 | Glyceric acid | X | | X |
| 57 | HMDB00191 | L-Aspartic acid | X | X | X |
| 58 | HMDB00094 | Citric acid | X | X | X |
| 59 | HMDB00694 | L-2-Hydroxyglutaric acid | X | | |
| 60 | HMDB00606 | D-2-Hydroxyglutaric acid | X | | |
| 61 | HMDB12275 | Phenylethylamine | | X | |
| 62 | HMDB00638 | Dodecanoic acid | X | X | |
| 63 | HMDB00187 | L-Serine | X | X | X |
| 64 | HMDB00641 | L-Glutamine | | X | X |
| 65 | HMDB00125 | Glutathione | X | X | X |
| 66 | HMDB00500 | 4-Hydroxybenzoic acid | X | | X |
| 67 | HMDB00669 | Ortho-Hydroxyphenylacetic acid | | | X |
| 68 | HMDB00148 | L-Glutamic acid | X | X | X |
| 69 | HMDB00696 | L-Methionine | X | X | X |
| 70 | HMDB00440 | 3-Hydroxyphenylacetic acid | X | | X |
| 71 | HMDB06524 | 3-Indoleacetonitrile | X | | X |
| 72 | HMDB00020 | p-Hydroxyphenylacetic acid | X | | X |
| 73 | HMDB00956 | Tartaric acid | X | | X |
| 74 | HMDB00510 | Aminoadipic acid | X | | X |
| 75 | HMDB00574 | L-Cysteine | | | X |
| 76 | HMDB00714 | Hippuric acid | | | X |
| 77 | HMDB00159 | L-Phenylalanine | X | X | X |
| 78 | HMDB00806 | Myristic acid | X | X | |
| 79 | HMDB00193 | Isocitric acid | | | X |
| 80 | HMDB02643 | 3-(3-Hydroxyphenyl)-3-hydroxypropanoic acid | X | | |
| 81 | HMDB00484 | Vanillic acid | | | X |
| 82 | HMDB00826 | Pentadecanoic acid | X | X | |
| 83 | HMDB00197 | Indoleacetic acid | | | X |
| 84 | HMDB02035 | 4-Hydroxycinnamic acid | X | | |
| 85 | HMDB03229 | Palmitoleic acid | X | X | |
| 86 | HMDB00840 | Salicyluric acid | | | X |
| 87 | HMDB00214 | Ornithine | X | X | X |
| 88 | HMDB00220 | Hexadecanoic acid | X | X | X |
| 89 | HMDB00182 | L-Lysine | X | X | X |
| 90 | HMDB02259 | Heptadecanoic acid | X | X | |
| 91 | HMDB00177 | L-Histidine | X | X | X |
| 92 | HMDB00673 | Linoleic acid | X | X | |
| 93 | HMDB00207 | Oleic acid | X | X | |
| 94 | HMDB00827 | Stearic acid | X | X | X |
| 95 | HMDB06116 | m-Hydroxyhippuric acid | | | X |
| 96 | HMDB00158 | L-Tyrosine | X | X | X |
| 97 | HMDB01043 | Arachidonic acid | X | X | |
| 98 | HMDB02212 | Eicosanoic acid | X | | |
| 99 | HMDB00929 | L-Tryptophan | X | X | X |
| 100 | HMDB13713 | N-acetyltryptophan | | X | X |
| 101 | HMDB02183 | Docosahexaenoic acid | X | X | |
| 102 | HMDB00944 | Docosanoic acid | X | | |
| 103 | HMDB00192 | L-Cystine | | | X |
| 104 | HMDB02003 | Tetracosanoic acid | X | | |

## Claims

1. Use of 2-methylbutyrate as a biomarker for detecting and/or quantifying mood disorders, improvements of the mood disorder status and/or the resulting emotional reaction of a subject.

2. Method for detecting and/or quantifying mood disorders, improvements of the mood disorder status and/or the resulting emotional reaction of a subject, comprising
- determining the level of 2-methylbutyrate in a body sample that has been obtained from a subject to be tested, and
- comparing the subject's 2-methylbutyrate level to a predetermined reference value,
wherein an increased 2-methylbutyrate level in the sample compared to the predetermined reference value indicates an improvement of the mood disorder/mood status and/or the resulting emotional reaction of the subject.

3. The method in accordance with claim2, wherein the predetermined reference value was obtained previously from the same subject.

4. The method in accordance with claim2, wherein the predetermined reference value is based on an average 2-methylbutyrate level in the same body fluid in a control population.

5. The method in accordance with one of claims 2 to 4, wherein the levels of the biomarkers in the sample and in the reference are determined by mass spectrometry.

6. The method in accordance with claim5, wherein the levels of the biomarkers in the sample and in the reference are determined by ultra-performance liquid or gas chromatography coupled to tandem mass spectrometry.

7. The method in accordance with one of claims 2 to 6, wherein the predetermined reference value is based on a 2-methylbutyrate level obtained from the same body fluid as the level of 2-methylbutyrate in a body sample obtained from a subject to be tested.

8. The method in accordance with one of claims 2 to 7, wherein the subject's predetermined reference value was obtained from a body sample that was collected from the subject before an intervention to treat or ameliorate a mood disorder status and/or the resulting emotional reaction started.

9. The method in accordance with one of claims 2 to 7, wherein the subject's predetermined reference value was obtained from a body sample that was collected from the subject during an intervention to treat or ameliorate a mood disorder status and/or the resulting emotional reaction, but at least one week, for example at least four weeks, before the body sample is obtained from the subject.

10. The method in accordance with one of claims 2 to 9, wherein an increase in the 2-methylbutyrate level in the sample compared to the predetermined reference value of at least 66% indicates an improvement of the mood disorder status and/or the resulting emotional reaction of the subject.

11. The method in accordance with one of claims 2 to 10, wherein the body sample is selected from the group consisting of feces, urine, blood, blood serum, and blood plasma.

12. The method in accordance with one of claims 2 to 11, wherein the method is for monitoring the progress of an intervention to treat or ameliorate a mood disorder status and/or the resulting emotional reaction in a subject, wherein the intervention comprises the administration of a probiotic.

13. The method in accordance with claim12, wherein the probiotic is *Bifidobacterium longum* NCC3001.

14. The method in accordance with one of claims 2 to 13, wherein the subject is a human or a companion animal such as a cat or a dog.

## Patentansprüche

1. Verwendung von 2-Methylbutyrat als einen Biomarker zum Erkennen und/oder Quantifizieren von affektiven Störungen, Besserungen des Status der affektiven Störung und/oder der resultierenden emotionalen Reaktion eines Subjekts.

2. Verfahren zum Erkennen und/oder Quantifizieren von affektiven Störungen, Besserungen des Status der affektiven Störung und/oder der resultierenden emotionalen Reaktion eines Subjekts, umfassend
- Bestimmen des Spiegels von 2-Methylbutyrat in einer Körperprobe, die von einem zu untersuchenden Subjekt erhalten wurde und
- Vergleichen des 2-Methylbutyratspiegels des Subjekts mit einem zuvor bestimmten Referenzwert,
wobei ein erhöhter 2-Methylbutyratspiegel in der Probe im Vergleich zu dem zuvor bestimmten Referenzwert eine Besserung der affektiven Störung/des affektiven Status und/oder der resultierenden emotionalen Reaktion des Subjekts angibt.

3. Verfahren nach Anspruch2, wobei der zuvor bestimmte Referenzwert vorher von demselben Subjekt erhalten wurde.

4. Verfahren nach Anspruch2, wobei der zuvor bestimmte Referenzwert auf einem durchschnittlichen 2-Methylbutyratspiegel in demselben Körperfluid in einer Kontrollpopulation basiert.

5. Verfahren nach einem der Ansprüche 2 bis 4, wobei die Spiegel der Biomarker in der Probe und in der Referenz durch Massenspektrometrie bestimmt werden.

6. Verfahren nach Anspruchs, wobei die Spiegel der Biomarker in der Probe und in der Referenz durch Ultra-Performance-Flüssigkeits- oder Gaschromatographie, gekoppelt an Tandem-Massenspektrometrie, bestimmt werden.

7. Verfahren nach einem der Ansprüche 2 bis 6, wobei der zuvor bestimmte Referenzwert auf einem 2-Methylbutyratspiegel basiert, der von demselben Körperfluid erhalten wird wie der Spiegel von 2-Methylbutyrat in einer Körperprobe, die von einem zu untersuchenden Subjekt erhalten wird.

8. Verfahren nach einem der Ansprüche 2 bis 7, wobei der zuvor bestimmte Referenzwert des Subjekts von einer Körperprobe erhalten wurde, die dem Subjekt entnommen wurde, bevor eine Intervention, um einen Zustand der affektiven Störung und/oder der resultierenden emotionalen Reaktion zu behandeln oder zu verbessern, begann.

9. Verfahren nach einem der Ansprüche 2 bis 7, wobei der zuvor bestimmte Referenzwert des Subjekts von einer Körperprobe erhalten wurde, die dem Subjekt entnommen wurde, während einer Intervention, um einen Zustand der affektiven Störung und/oder der resultierenden emotionalen Reaktion zu behandeln oder verbessern, jedoch mindestens eine Woche, zum Beispiel mindestens vier Wochen, bevor die Körperprobe von dem Subjekt erhalten wird.

10. Verfahren nach einem der Ansprüche 2 bis 9, wobei eine Erhöhung des 2-Methylbutyratspiegels in der Probe im Vergleich zu dem zuvor bestimmten Referenzwert von mindestens 66 % eine Besserung des Zustands der affektiven Störung und/oder der resultierenden emotionalen Reaktion des Subjekts angibt.

11. Verfahren nach einem der Ansprüche 2 bis 10, wobei die Körperprobe aus der Gruppe ausgewählt ist, bestehend aus Fäkalien, Urin, Blut, Blutserum und Blutplasma.

12. Verfahren nach einem der Ansprüche 2 bis 11, wobei das Verfahren zum Überwachen des Fortschritts einer Intervention, um einen Zustand der affektiven Störung und/oder der resultierenden emotionalen Reaktion in einem Subjekt zu behandeln oder verbessern, dient, wobei die Intervention die Verabreichung eines Probiotikums umfasst.

13. Verfahren nach Anspruch12, wobei das Probiotikum *Bifidobacterium longum* NCC3001 ist.

14. Verfahren nach einem der Ansprüche 2 bis 13, wobei das Subjekt ein Mensch oder ein Haustier wie etwa eine Katze oder ein Hund ist.

## Revendications

1. Utilisation du 2-butyrate de méthyle en guise de biomarqueur pour la détection et/ou la quantification des troubles de l'humeur, des améliorations de l'état de trouble de l'humeur et/ou de la réaction émotionnelle résultante d'un sujet.

2. Procédé pour la détection et/ou la quantification des troubles de l'humeur, des améliorations de l'état de trouble de l'humeur et/ou de la réaction émotionnelle résultante d'un sujet, comprenant
- la détermination du taux de 2-butyrate de méthyle dans un échantillon corporel qui a été obtenu à partir d'un sujet à tester, et
- la comparaison du taux de 2-butyrate de méthyle du sujet avec une valeur de référence prédéterminée,
dans lequel un taux de 2-butyrate de méthyle augmenté dans l'échantillon par comparaison avec la valeur de référence prédéterminée indique une amélioration du trouble de l'humeur/état de l'humeur et/ou de la réaction émotionnelle résultante du sujet.

3. Procédé selon la revendication 2, dans lequel la valeur de référence prédéterminée a été précédemment obtenue à partir du même sujet.

4. Procédé selon la revendication 2, dans lequel la valeur de référence prédéterminée est fonction d'un taux moyen de 2-butyrate de méthyle dans le même fluide corporel dans une population témoin.

5. Procédé selon l'une des revendications 2 à 4, dans lequel les taux des biomarqueurs dans l'échantillon et dans la référence sont déterminés par spectrométrie de masse.

6. Procédé selon la revendication 5, dans lequel les taux des biomarqueurs dans l'échantillon et dans la référence sont déterminés par chromatographie liquide ou gazeuse à ultra-performance couplée à une spectrométrie de masse en tandem.

7. Procédé selon l'une des revendications 2 à 6, dans lequel la valeur de référence prédéterminée est fonction d'un taux de 2-butyrate de méthyle obtenu à partir du même fluide corporel en guise de taux de 2-butyrate de méthyle dans un échantillon corporel obtenu à partir d'un sujet à tester.

8. Procédé selon l'une des revendications 2 à 7, dans lequel la valeur de référence prédéterminée du sujet a été obtenue à partir d'un échantillon corporel qui a été recueilli à partir du sujet avant une intervention pour traiter ou améliorer un état de trouble de l'humeur et/ou la réaction émotionnelle résultante initiée.

9. Procédé selon l'une des revendications 2 à 7, dans lequel la valeur de référence prédéterminée du sujet a été obtenue à partir d'un échantillon corporel qui a été recueilli à partir du sujet pendant une intervention pour traiter ou améliorer un état de trouble de l'humeur et/ou la réaction émotionnelle résultante, mais au moins une semaine, par exemple au moins quatre semaines, avant que l'échantillon corporel soit obtenu à partir du sujet.

10. Procédé selon l'une des revendications 2 à 9, dans lequel une augmentation du taux de 2-butyrate de méthyle dans l'échantillon par comparaison avec la valeur de référence prédéterminée d'au moins 66 % indique une amélioration de l'état de trouble de l'humeur et/ou de la réaction émotionnelle résultante du sujet.

11. Procédé selon l'une des revendications 2 à 10, dans lequel l'échantillon corporel est choisi dans le groupe constitué de fèces, urine, sang, sérum sanguin, et plasma sanguin.

12. Procédé selon l'une des revendications 2 à 11, dans lequel le procédé est destiné à surveiller la progression d'une intervention pour traiter ou améliorer un état de trouble de l'humeur et/ou la réaction émotionnelle résultante chez un sujet, dans lequel l'intervention comprend l'administration d'un probiotique.

13. Procédé selon la revendication 12, dans lequel le probiotique est *Bifidobacterium longum* NCC3001.

14. Procédé selon l'une des revendications 2 à 13, dans lequel le sujet est un humain ou un animal de compagnie tel qu'un chat ou un chien.
